# EUROPEAN PATENT SPECIFICATION

(11) **EP 1 502 593 B1**
(45) Date of publication and mention of the grant of the patent: **14.02.2007**
(21) Application number: 04017477.3
(22) Date of filing: 23.07.2004
(51) Int. Cl.: A61K 31/58, A61P 11/00

(54) **The use of Stanozolol for the preparation of anti-inflammatory and anti-degenerative drugs**
Verwendung von Stanozolol zur Herstellung von entzündungshemmenden und antidegenerativen Arzneimitteln
Utilisation de Stanozolole pour la préparation de médicaments anti-inflammatoires et anti-dégénératifs

(30) Priority: 29.07.2003 IT mi20031553; 20.02.2004 IT mi20040290
(43) Date of publication of application: 02.02.2005
(73) Proprietor: ACME S.R.L., 42025 Cavriago (RE) (IT)
(72) Inventor: Dondi, Maurizio, 42025 Cavriago (RE) (IT); Predieri, Paolo, 42025 Cavriago (RE) (IT)
(74) Representative: Minoja, Fabrizio

(56) References cited:
- EP-A- 0 324 037
- WO-A-98/03180
- US-A- 6 107 289
- US-A1- 2003 022 877
- US-A1- 2003 139 362
- BELCH J J F ET AL: "The effect of increasing fibrinolysis in patients with rheumatoid arthritis: A double blind study of stanozolol" QUARTERLY JOURNAL OF MEDICINE 1986 UNITED KINGDOM, vol. 58, no. 225, 1986, pages 19-27, XP008037619
- STEPÁN J ET AL: "[Stromba in disorders of the locomotion apparatus]" MEDIZINISCHE KLINIK. 22 SEP 1967, vol. 62, no. 38, 22 September 1967 (1967-09-22), pages 1470-1474, XP008037622 ISSN: 0025-8458
- BURNAND K ET AL: "Venous lipodermatosclerosis: treatment by fibrinolytic enhancement and elastic compression." BRITISH MEDICAL JOURNAL. 5 JAN 1980, vol. 280, no. 6206, 5 January 1980 (1980-01-05), pages 7-11, XP008037621 ISSN: 0007-1447
- KERDEL F A: "Inflammatory ulcers" JOURNAL OF DERMATOLOGIC SURGERY AND ONCOLOGY 1993 UNITED STATES, vol. 19, no. 8, 1993, pages 772-778, XP008037630 ISSN: 0148-0812
- FALANGA VINCENT ET AL: "Stimulation of collagen synthesis by the anabolic steroid stanozolol" JOURNAL OF INVESTIGATIVE DERMATOLOGY, vol. 111, no. 6, December 1998 (1998-12), pages 1193-1197, XP001075053 ISSN: 0022-202X
- YERSIN A G ET AL: "The effects of Bordetella avium infection on elastin and collagen content of turkey trachea and aorta." POULTRY SCIENCE. NOV 1998, vol. 77, no. 11, November 1998 (1998-11), pages 1654-1660, XP008037625 ISSN: 0032-5791

## Description

The present invention relates to the use of stanozolol for the preparation of pharmaceutical compositions for the treatment of animal disorders supported or complicated by inflammatory and/or degenerative causes, namely canine tracheal collapse.

Stanozolol is a steroid hormone, which has long been used in human and veterinary medicine for its anabolic effects.

Anabolic agents are based on steroid molecules derived from testosterone; these molecules are able to stimulate the anabolism of human and animal cells.

However, their use in medicine is limited by the adverse effects typical of these molecules, such as a masculinisation or virilisation, and increased aggressiveness.

In order to extend the possibilities of clinical use of anabolic steroids, pharmacological researchers have endeavoured to obviate these adverse effects by synthesising new molecules which, while maintaining marked anabolic properties, are accompanied by low masculinising effects. This object has been achieved to a good extent, and a number of synthetic molecules with a satisfactory anabolic-androgenic ratio, including stanozolol, are currently available for treatment.

Anabolic agents are currently used to stimulate the appetite, improve blood crasis, haemopoiesis and tissue repair following traumas and burns, and to increase the muscle mass of asthenic and debilitated patients.

In orthopaedics, anabolic agents are commonly used to improve the calcification of bone tissue, such as in the case of osteoporosis or after fractures.

In addition to its anabolic effect, stanozolol has been found to exert a powerful anti-inflammatory effect, comparable to that performed by cortisol, the most classic corticosteroid hormone physiologically produced by the bodies of the higher mammals, together with an anti-degenerative effect on the tissues.

The anti-inflammatory effect of stanozolol has been demonstrated *in vitro* on chondrocyte cell cultures by measuring the production of nitric oxide (NO).

The anti-degenerative effect has been demonstrated *in vitro* by measuring the tissue production of cytokines belonging to the Insulin-Like Growth Factor (IGF 1) family.

### Anti-inflammatory effect

Nitric oxide is scientifically recognised as being a reliable parameter for evaluation of the inflammatory state of tissues.

Chondrocyte cell cultures were implanted; part of the culture was then stimulated with stanozolol, while the remainder was cultivated in the absence of pharmacological stimulation, and examined as reference sample.

The inhibition of nitric oxide (NO) production in the chondrocyte cell cultures stimulated with stanozolol was statistically greater than that recorded in the control chondrocyte cultures, which means that nitric oxide production was lower in the stimulated than the control cultures (mean values "I" in micromoles of nitric oxide: 2.36 as against 3.23). The activity proved similar or superior to that of powerful known anti-inflammatories such as hydrocortisone and dexamethasone. In the same test, testosterone substantially failed to influence nitric oxide production, and consequently lacks anti-inflammatory effects.

### Anti-degenerative effect

The cytokines belonging to the Insulin-Like Growth Factor (IGF 1) family act by promoting the synthesis of collagen, proteoglycans and other extracellular cartilage matrix proteins, and therefore play a crucial part in tissue repair and protection.

Chondrocyte cell cultures were implanted for this purpose; part of the culture was stimulated with stanozolol, dexamethasone and testosterone, while the remainder was cultivated as control, without any pharmacological stimulation. IGF 1 production was measured on all the cell cultures, and it was found that the chondrocyte cultures stimulated with stanozolol synthesised significantly larger amounts than the other two compounds and the control chondrocyte cultures. More specifically, the mean IGF1 production values amounted to 5.85 mcg/ml in the chondrocyte cultures stimulated with stanozolol, 3.89 mcg/ml in those treated with dexamethasone, 1.64 mcg/ml in those treated with testosterone, and 1.63 mcg/ml in the control cultures.

On a percentage basis, mean values "I" correspond to 358.85 in the cultures stimulated with stanozolol, 198.25 for dexamethasone, 100.31 for testosterone and 100.20 for the control.

Consequently, stanozolol can be used in all disorders caused, supported or influenced in their development by inflammatory and/or degenerative etiological components as well as disorders which obtain clinical benefits not only from the classic stimulating effect on cell replication (this action being more or less common to all anabolic agents), but above all by the anti-inflammatory and anti-degenerative action of stanozolol. Stanozolol can play a useful part in these disorders by interrupting the vicious circle of inflammation - leukocyte recruitment - release of tissue-damaging and proinflammatory enzymes - tissue degeneration - inflammation.

Stanozolol can be useful in degenerative disorders, especially those in which stimulation of the production of insulin-like growth factor 1 is advantageous, as it interrupts the sequence of physiopathological events that cause the tissues to degenerate and stimulates tissue healing, thus promoting functional recovery of the patient biological activities.

The invention thus relates to the use of stanozolol for the preparation of pharmaceutical compositions for the treatment of canine tracheal collapse.

Tracheal collapse, a respiratory disorder which mainly affects small dogs, is a degenerative disorder caused by loss of cartilage matrix from the tracheal rings.

It has been found that stanozolol effectively treats this disorder thanks to the dose-dependent increase in collagen production, mediated by synthesis of Transforming Growth Factor (TGF-β1) which has a favourable mitogenic action on the mesenchymal cells, promotes the production of integrin, and reduces the synthesis of proteases which cause deterioration of the extracellular protein matrix.

The pharmaceutical compositions according to claim 1 can be administered by oral, parenteral (including mesotherapy), intra-articular or topical routes.

By way of example, the preferable doses are as follows:
oral route: 0.0003 - 30 mg/kg of weight;
parenteral route: 0.001 - 100 mg/kg of weight;

These doses are appropriate for a single administration; the product can be administered one or more times a day, even in long-term treatments or throughout the patient's life. These pharmaceutical compositions will be prepared according to conventional methods well known in pharmaceutical technology, like those described in "Remington's Pharmaceutical Handbook", Mack Publishing Co., N.Y., USA, together with suitable excipients commonly used in pharmaceutical technology.

## Claims

1. The use of stanozolol for the preparation of pharmaceutical compositions for the treatment of canine tracheal collapse.

## Patentansprüche

1. Verwendung von Stanozolol für die Bereitstellung von pharmazeutischen Zusammensetzungen für die Behandlung des Trachealkollapses in Hunden.

## Revendications

1. Utilisation de stanozolol pour la préparation de compositions pharmaceutiques pour le traitement du collapsus trachéal canin.
